# EUROPÄISCHE PATENTSCHRIFT

(11) **EP 1 255 113 B1**
(45) Veröffentlichungstag und Bekanntmachung des Hinweises auf die Patenterteilung: **19.05.2004**
(21) Anmeldenummer: 02009699.6
(22) Anmeldetag: 29.04.2002
(51) Int. Cl.: G01N 33/74

(54) **Verfahren zur Testung des hormonellen Effekts von Substanzen**
Assay for the determination of the hormonal effect of compounds
Méthode de détermination de l'effet hormonal de molécules

(30) Priorität: 04.05.2001 DE 10121710; 13.12.2001 DE 10161325
(43) Veröffentlichungstag der Anmeldung: 06.11.2002
(73) Patentinhaber: Jenapharm GmbH & Co. KG, 07745 Jena (DE)
(72) Erfinder: Obendorf, Maik, Dr., 99423 Weimar (DE); Schröder, Jens, Dr., 07743 Jena (DE); Wolf, Siegmund, Dr., 07745 Jena (DE)
(74) Vertreter: Störle, Christian, Dr.

(56) Entgegenhaltungen:
- US-A- 5 506 102
- DING XIU FEN ET AL: "Nuclear receptor-binding sites of coactivators glucocorticoid receptor interacting protein 1 (GRIP1) and steroid receptor coactivator 1 (SRC-1): Multiple motifs with different binding specificities" MOLECULAR ENDOCRINOLOGY, BALTIMORE, MD, US, Bd. 12, Nr. 2, Februar 1998 (1998-02), Seiten 302-313, XP001040351 ISSN: 0888-8809
- MCKENNA N J ET AL: "NUCLEAR RECEPTOR COREGULATORS: CELLULAR AND MOLECULAR BIOLOGY" ENDOCRINE REVIEWS, BALTIMORE, MD, US, Bd. 20, Nr. 3, Juni 1999 (1999-06), Seiten 321-344, XP002921971
- DATABASE EBI [Online] retrieved from EBI, accession no. Q9UHS5 Database accession no. Q9UHS5 XP002207652
- STENOIEN D L ET AL: "LIGAND-MEDIATED ASSEMBLY AND REAL-TIME CELLULAR DYNAMICS OF ESTROGEN RECEPTOR ALPHA-COACTIVATOR COMPLEXES IN LIVING CELLS" MOLECULAR AND CELLULAR BIOLOGY, AMERICAN SOCIETY FOR MICROBIOLOGY, WASHINGTON, US, Bd. 21, Nr. 13, Juli 2001 (2001-07), Seiten 4404-4412, XP001058122 ISSN: 0270-7306

## Beschreibung

Die Erfindung bezieht sich auf ein Verfahren zur Testung des hormonellen Effekts von Substanzen, ein Verfahren zur Bestimmung von Störungen im Co-Modulationsmechanismus von nukleären Rezeptoren sowie hierzu geeignete Mittel, insbesondere den Co-Aktivator ARAP11 für den humanen Androgenrezeptor und für andere nukleäre Rezeptoren sowie dafür kodierende DNA.

Bei der Beurteilung von Substanzen für eine mögliche pharmazeutische Anwendung ist es allgemein üblich, diese Substanzen auf eine eventuelle hormonelle Wirkung, insbesondere auf eine möglicherweise vorhandene androgene oder antiandrogene Aktivität, zu prüfen. Bei der Verabreichung pharmakologisch wirksamer Substanzen sind Kenntnisse über hormonelle Effekte, insbesondere androgene oder antiandrogene Effekte, dieser Substanzen in manchen Fällen von Bedeutung, da sie beim Patienten unerwünschte Nebenwirkungen hervorrufen können. Zur Prüfung der hormonellen Wirkung von Substanzen kommen insbesondere Verfahren zum Einsatz, bei denen die Fähigkeit der Substanzen gemessen wird, an die Hormonrezeptoren zu binden und deren Transkriptionsaktivität zu aktivieren.

Kenntnisse über hormonelle Effekte von Substanzen sind aber nicht nur bei potentiellen Pharmaka von Interesse, sondern auch bei nicht-pharmazeutischen Substanzen, da von vielen, in der Umwelt vorhandenen Substanzen angenommen wird, dass sie bei Teilen der Bevölkerung eine androgene oder antiandrogene bzw. estrogene oder antiestrogene Aktivität aufweisen können. Möglicherweise wird dadurch eine unerwünschte, schädliche Wirkung hervorgerufen.

Es besteht also ein ganz erhebliches Bedürfnis für ein Verfahren und für ein für die Durchführung des Verfahrens geeignetes Mittel, mit dem in zuverlässiger, empfindlicher, einfacher, kostengünstiger und schneller Weise eine Aussage über den hormonellen Effekt von Substanzen getroffen werden kann. Die bisher bekannten Verfahren genügen dem nicht.

Der vorliegenden Erfindung liegt deshalb die Aufgabe zu Grunde, ein Verfahren und dazu geeignete Mittel bereitzustellen, mit dem in zuverlässiger, empfindlicher, einfacher, kostengünstiger und schneller Weise eine Aussage über den hormonellen Effekt der zu testenden Substanzen getroffen werden kann.

Erfindungsgemäß wird dies in überraschender Weise erreicht durch ein Verfahren zur Testung des hormonellen Effekts, insbesondere des androgenen oder antiandrogenen Effekts, von Substanzen, bei dem
a) Zellen, die mit zwei Vektoren transfektiert sind, wobei der eine Vektor DNA, die für ein nukleäres Rezeptor-Protein oder ein Fragment davon, insbesondere ein humanes nukleäres Rezeptor-Protein oder ein Fragment davon, kodiert, und der andere Vektor DNA enthält, die für einen Co-Modulator oder ein Fragment davon kodiert, der Substanz ausgesetzt werden und
b) die Transkriptionsaktivität, die der nukleäre Rezeptor oder dessen Fragment in Anwesenheit des Co-Modulators oder dessen Fragment auslöst, und/oder der Einfluss der Substanz auf die Interaktion zwischen dem Rezeptor oder dessen Fragment und dem Co-Modulator oder dessen Fragment durch Protein-Protein-Interaktion oder Protein-Protein-DNA-Interaktion gemessen wird, wobei der Co-Modulator ARAP11 ist, das die in SEQ ID Nr. 1 angegebene Aminosäuresequenz aufweist.

Es wurde überraschenderweise gefunden, dass mit dem erfindungsgemäßen Verfahren in zuverlässiger, empfindlicher, einfacher, schneller und kostengünstiger Weise getestet werden kann, ob Substanzen, die beispielsweise umweltrelevant oder pharmakologisch von Interesse sein können, einen hormonellen Effekt, insbesondere einen androgenen oder antiandrogenen Effekt, ausüben.

Im erfindungsgemäßen Verfahren werden mit einem Vektor transformierte Zellen eingesetzt, wobei die Vektoren DNA aufweisen, die für ein nukleäres Rezeptor-Protein oder ein Fragment davon kodiert.

Die Superfamilie der nukleären Rezeptoren (NRs), zu der mehr als 50 verschiedene Proteine gehören, ist eine Gruppe verwandter Transkriptionsfaktoren, die die Transkription des jeweiligen Zielgens als Reaktion auf spezifische Liganden, z. B. Hormone, steuern. Die Familie kann nach bestimmten Charakteristika, wie z.B. Dimerisationsstatus, Art des Liganden oder Struktur des DNA-Reaktionselements, in mehrere Subfamilien unterteilt werden (Beato et al., 2000, Human Reproduct. Update, 6, 225-236). Charakteristisches Merkmal der NRs ist die übereinstimmende Struktur der funktionellen Domäne (mit den Bezeichnungen A bis F) mit einer stark variablen, nur schwach konservierten N-terminalen Region mit autonomer konstitutiver Aktivierungsfunktion (AF-1), einer stark konservierten DNA-Bindungsdomäne (DBD), die für die Erkennung von speziellen DNA-Reaktionselementen verantwortlich ist und aus zwei Zinkfinger-Motiven besteht, einer variablen Scharnierdomäne und einer konservierten multifunktionalen C-terminalen Ligandenbindungsdomäne (LBD) mit Dimerisations- und Liganden-abhängiger Transaktivierungsfunktion (AF-2). Im Anschluss daran folgt die am weitesten C-terminal gelegene Region, deren Funktion nicht bekannt ist und die bei Rezeptoren wie z.B. PR (Progesteron-Rezeptor), PPAR (Peroxisomproliferator-aktivierter Rezeptor) und RXR (Retinoid-X-Rezeptor) fehlt (Mangelsdorf & Evans, 1995; Cell, 83, 841-850; Robyr et al., 2000, Mol. Endocrinol., 14, 329-347). Für einige NRs (z.B. den Androgen-Rezeptor (AR)) wurde nachgewiesen, dass die N-terminale Region in der Lage ist, mit der C-terminalen Region zu interagieren (Brinkmann et al., 1999, J. Steroid Biochem. and Mol. Biol., 69, 307-313). Steroidhormonrezeptoren wie z.B. Estrogen- (ER), Progesteron- (PR), Glukokortikoid- (GR), Mineralokortikoid- (MR) und Androgenrezeptoren (AR) binden steroidale Liganden, die sich von Pregnenolon ableiten, wie die Progestine, die Estrogene, die Glukokortikoide und die Mineralokortikoide, sowie Androgene. Die Ligandenbindung aktiviert den Rezeptor und steuert die Expression entsprechender Zielgene.

Wie vorstehend ausgeführt wurde, werden in Schritt a) des erfindungsgemäßen Verfahrens Zellen verwendet, die einen Vektor enthalten, der für den Co-Modulator ARAP11 oder ein Fragment davon kodierende DNA aufweist.

Die Co-Modulatoren sind eine Klasse von Proteinen, die bei der Aktivierung (Co-Aktivatoren) bzw. Repression (Co-Repressoren) der Gentranskription als Brückenmoleküle zwischen dem Transkriptionsinitiationskomplex und den NRs dienen (McKenna et al., 1999, Endocr. Rev., 20, 321-347). Ein Co-Aktivator muss fähig sein, die Rezeptorfunktion zu verstärken und in Anwesenheit eines Agonisten mit der Aktivierungsdomäne von NRs direkt zu interagieren. Er muss auch mit dem basalen Transkriptionsapparat interagieren, und schließlich darf er nicht selbst die basale Transkriptionsaktivität verstärken. Die meisten Co-Modulatoren interagieren mit Hilfe eines oder mehrerer LXXLL-Motiv(en) (NR-Boxes) mit der AF-2-Domäne von NRs, jedoch wurden auch einige Co-Modulatoren beschrieben, die mit anderen NR-Regionen interagieren (Ding et al., 1998, Mol. Endocrinol., 12, 302-313). Ferner wurden viele Co-Modulatoren identifiziert, die in ähnlicher Weise mit mehreren verschiedenen NRs interagieren, so dass günstigerweise der Spezifizitätsgrad jedes Co-Modulators getestet werden sollte.

Im erfindungsgemäßen Verfahren wird der mit ARAP11 bezeichnete Co-Modulator oder das die Aminosäuren 813 bis 1390 aufweisende Fragment von ARAP11 eingesetzt. Die cDNA-Sequenz bzw. die 1390 Aminosäuren aufweisende Aminosäurensequenz des Co-Modulators ARAP11 ist in SEQ ID No. 1 bzw. SEQ ID No. 2 dargestellt. Bei Verwendung dieser Proteine kann in besonders zuverlässiger, empfindlicher, einfacher, kostengünstiger und schneller Weise das erfindungsgemäße Verfahren durchgeführt werden. Ferner weisen die ARAP11 Fragmente, insbesondere das die Aminosäuren 813 bis 1390 aufweisende Fragment von ARAP11, den Vorteil auf, daß sie leichter handhabbar und klonierbar sind, aber noch die funktiellen Eigenschaften von ARAP 11 aufweisen.

Bei ARAP11 handelt es sich um einen Co-Aktivator für den humanen Androgenrezeptor und andere nukleäre Rezeptoren, der die Interaktion zwischen einem Androgen und dem Rezeptor verstärkt. Ein Teil der Sequenz von ARAP11 ist bereits als Pro2000 in der Genbank XM 005253 beschrieben; allerdings ist dort keine Funktion angegeben. Im Vergleich zu der bereits aus der Genbank bekannten Sequenz wurde nun festgestellt, daß die Aminosäuresequenz von ARAP 11 größer als die bekannte Sequenz ist; es weist am N-terminalen Bereich zusätzliche Aminosäuren auf. Ferner konnte eine Interaktion zwischen nukleären Rezeptoren, insbesondere AR, einerseits und ARAP11 andererseits sowie eine Verstärkung der AR-vermittelten Transaktivierung festgestellt werden. ARAP11 ist ein Protein, das als Co-Mediator fungiert, indem es nach Bindung von Steroiden an den nukleären Rezeptor den Transkriptionseffekt verstärkt oder reprimiert und darüber hinaus die Bindung und Aktivierung des nukleären Rezeptors an Moleküle fördert, denen früher keine hormonelle Wirkung zugeschrieben wurde.

Das Protein ARAP11 stellt einen Co-Aktivator für den Androgenrezeptor und weitere nukleäre Rezeptoren dar, wie Estrogenrezeptor α, Estrogenrezeptor β, Progesteronrezeptor A, Progesteronrezeptor B, Glukokortikoidrezeptor, Mineralokortikoidrezeptor, Schilddrüsenhormonrezeptor, Vitamin-D-Rezeptor, Peroxisomproliferator-aktivierter Rezeptor, Retinsäurerezeptor, Retinoid-X-Rezeptor und Orphan-Rezeptoren; im erfindungsgemäßen Verfahren werden diese Rezeptoren bevorzugt eingesetzt, da damit die vorstehenden Vorteile des erfindungsgemäßen Verfahrens besonders günstig erreicht werden können.

Im erfindungsgemäßen Verfahren können auch Vektoren, die für Fragmente vorstehender Proteine kodieren, eingesetzt werden. Unter dem Ausdruck "Fragmente" im Zusammenhang mit vorstehenden Proteinen werden solche verstanden, die eine Aminosäure oder mehrere Aminosäuren weniger als die Proteine in voller Länge und noch die funktionellen Eigenschaften eines nukleären Rezeptors oder eines Co-Modulators aufweisen.

Wie bereits vorstehend ausgeführt wurde, werden im erfindungsgemäßen Verfahren in Schritt a) Zellen eingesetzt, die mit zwei Vektoren transfektiert sind, die für spezielle Proteine kodierende DNA enthalten. Diese Zellen sind also in der Lage, diese beiden unterschiedlichen Proteine zu exprimieren.

Vorzugsweise sind die Zellen etablierte Zelllinien und/oder eukaryotische Zellen, insbesondere Prostatazellen, Nervenzellen, Gliazellen, Fibroblasten, Blutzellen, Osteoblasten, Osteoklasten, Hepatozyten, Epithelzellen oder Muskelzellen. Mit den etablierten Zelllinien kann das erfindungsgemäße Verfahren besonders kostengünstig und schnell durchgeführt werden. Bei Verwendung eukaryotischer Zellen, insbesondere vorstehend aufgeführter eukaryotischer Zellen, können mit dem erfindungsgemäßen Verfahren vorteilhafterweise besonders aussagekräftige Ergebnisse erhalten werden.

In einer bevorzugten Ausführungsform des erfindungsgemäßen Verfahrens werden eukaryotische Expressionsvektoren eingesetzt, z.B. pCMX oder pSG5. Bei Verwendung dieser Vektoren, insbesondere in Verbindung mit vorstehenden etablierten Zelllinien und/oder eukaryotischen Zellen, kann das erfindungsgemäße Verfahren besonders günstig und schnell durchgeführt werden, und es werden besonders aussagekräftige Ergebnisse erhalten.

Der Fachmann kennt Verfahren und dazu notwendige Materialien, um die für die vorstehenden Proteine kodierende DNA in einen Vektor zu inserieren, diesen dann in die Zellen einzubringen und die so erhaltenen Zellen unter geeigneten Kulturbedingungen zu kultivieren, damit sie diese Proteine exprimieren können.

Gemäß Schritt b) des erfindungsgemäßen Verfahrens kann die Transkriptionsaktivität gemessen werden, die der nukleäre Rezeptor oder dessen Fragment in Anwesenheit des Co-Modulators oder dessen Fragment auslöst. Dies kann beispielsweise durch Detektion eines Reportergens erfolgen.

Reportergene sind Gene oder Genfragmente, die mit anderen Genen oder regulatorischen Sequenzen gekoppelt werden, um die Aktivität dieser Sequenzen nachweisbar zu machen. Reportergene erzeugen Genprodukte, die möglichst einfach nachweisbar sind, z. B. photometrisch durch Farbreaktionen. Häufig verwendete Reportergene sind das Gen für β-Galactosidase, das Gen für die alkalische Phosphatase, das Gen für Chloramphenicol-Acetyltransferase, das Gen für die Catechol-Dioxygenase, das Gen für das "green fluorescent protein" sowie verschiedene Luciferase-Gene, die die Zellen zum Leuchten bringen können.

Solche Reportergene können ebenfalls mittels Vektoren, insbesondere eukaryotischer Expressionsvektoren, in die Zellen eingebracht werden. Beispiel eines Vektors, der für ein Reportergen kodierende DNA enthält, ist der Vektor MMTV-Luciferase, der zur Messung der androgenen Wirkung von Substanzen eingesetzt wird.

Substanzen mit einem hormonellen Effekt, insbesondere einem androgenen/antiandrogenen Effekt, sind dann an der erhöhten bzw. verminderten Aktivität des Reportergens erkennbar.

Die Messung des Einflusses der Testsubstanz auf die Interaktion zwischen dem Rezeptor oder dessen Fragment und dem Co-Modulator oder dessen Fragment kann auch durch Bestimmung der Protein-Protein-Interaktion, z. B. durch Doppelt-Hybrid-Systeme, Immunpräzipitation, GST-Pull-down-Assays, FRET-Analayse und ABCD-Assays, sowie durch Bestimmung der Protein-Protein-DNA-Interaktion, wie durch Gelretardationsassays, erfolgen.

Es wurde ferner gefunden, dass ARAP11 sehr gut als Indikator androgenbedingter Erkrankungen, die zum Teil erst im Alter auftreten, verwendet werden kann. Relevante androgenbedingte Erkrankungen, wie z.B. Prostatakrebs, erektile Dysfunktion, Infertilität, Glatzenbildung, Akne oder Hypogonadismus, sowie Androgenresistenzsyndrome, wie z.B. die testikuläre Feminisierung, beruhen auf Defekten im Co-Modulationsmechanismus zwischen AR und ARAP11. Eine Möglichkeit bei Patienten mit derartigen Störungen besteht somit in der Messung der relativen Konzentrationen von AR und ARAP11. Diese Messung erfolgt günstigerweise in Körperflüssigkeiten, Körperzellen oder Körpergewebe extrakorporal. Dies ist möglich durch Anwendung quantitativer Verfahren zur Messung der relativen Menge beider Moleküle bei dem jeweiligen Patienten, bei denen beispielsweise Antikörper sowohl gegen AR als auch gegen ARAP11 oder Nukleinsäuresonden gegen deren mRNA eingesetzt werden können. Es gibt mehrere Verfahren zur Messung dieser komparativen Raten, die dem Fachmann bekannt sind; auch kennt er hierzu geeignete Materialien und Vorrichtungen, wie Radioimmunassay, ELISA-Test, Immunfärbung, RT-PCR, Western-Blot, Northem-Blot, DNA-Chip oder Protein-Chip. Darüber hinaus ist es möglich, mit Hilfe der ARAP11-cDNA in üblicher Weise Sonden für ein PCR-Assay zu konstruieren, mit dem sich bei bestimmten Patienten Mutationen der normalen DNA-Sequenz nachweisen oder Transkripte für den Northern Blot Assay bzw. eine DNA für In-situ-Hybridisierungsassays generieren lassen.

Das gemessene Verhältnis von AR zu ARAP11 kann dabei größer oder kleiner als das bei Gesunden sein. Der bei Gesunden vorliegende Normalwert kann in einfacher Weise beispielsweise dadurch bestimmt werden, dass das Verhältnis von AR zu ARAP11 bei einer Vielzahl von gesunden Probanden gemittelt wird. Durch den Vergleich des Normalwertes mit dem ermittelten Verhältnis von AR zu ARAP11 bei dem zu untersuchenden Patienten kann festgestellt werden, ob der Wert für das ermittelte Verhältnis größer oder kleiner als der Normalwert ist.

Da die Konzentration von ARAP11 und/oder AR im Gewebe unterschiedlich sein kann, z.B. ist die Konzentration von ARAP11 in Hoden sehr groß, wohingegen sie in Leber, Herz, Thymus und Prostata geringer ist, sind die unterschiedlichen Gewebekonzentrationen für die Auswertung zu berücksichtigen, d.h. der Testwert und der Normalwert sollen vom gleichen Gewebe stammen.

Eine andere Möglichkeit zur Bestimmung von Defekten im Co-Modulationsmechanismus zwischen AR und ARAP11 kann darin bestehen, nur die Konzentration von ARAP11 zu messen, wobei man dabei davon ausgeht, daß die AR-Konzentration zumindest annäherend konstant ist. Ist hierbei eine geringere als die normale ARAP11-Konzentration gemessen worden, heißt dies, daß sich das Verhältnis von AR zu ARAP11 verschoben hat, was wiederum als Hinweis auf eine Störung im Co-Modulationsmechanismus hinweist.

Es is also möglich, mit einer ARAP11 spezifischen Sonde Änderungen in der ARAP11-Expression und damit im Verhältnis zu AR zu bestimmen. Solche Änderungen können bei verschiedenen Krankheitsbildern ursächlich involviert sein oder als Folgeerscheinung auftreten.

Derartigen Messungen des AR/ARAP11-Verhältnisses bzw. von ARAP11 liegt die überraschende und auf die Auffindung und Charakterisierung von ARAP11 beruhende Erkenntnis zugrunde, dass beispielsweise ein Androgenresistenzsyndrom auf einer Störung des Gleichgewichts zwischen AR- und ARAP11-Prävalenz in den Zielzellen beruhen kann. Zuviel ARAP11 könnte zu einer Überempfindlichkeit des AR-Systems führen, so dass es auf Moleküle reagiert, die normalerweise keinen androgenen Effekt haben. Umgekehrt führt das Fehlen oder eine Malfunktion von ARAP11 auf allen Ebenen zur Androgenresistenz. Der Nachweis von zu viel ARAP11 bei einem Patienten spräche für die Anwendung von Mitteln zur Down-Regulation, wie z.B. Antisense- oder ähnlichen Medikamenten, um unter klinischen Bedingungen den ARAP11-Titer bei dem jeweiligen Patienten zu reduzieren. Dasselbe kann durch Moleküle erreicht werden, die in der Lage sind, die Interaktion zwischen AR und ARAP11 zu hemmen. Hat ein Patient zu wenig ARAP11, kann man ihm ARAP11-cDNA, -Protein oder -DNA über verschiedene an sich bekannte Mechanismen zuführen, um auf diese Weise den Titer des aktiven ARAP11 zu erhöhen. Möglich ist auch eine Anhebung der Konzentration oder der Aktivität von ARAP11 durch niedrigmolekulare Arzneimittel oder durch Stimulation der Eigensynthese mit Hilfe spezifischer ARAP11-Promoter-Proteine.

Wie aus den vorstehenden Ausführungen zu den erfindungsgemäßen Verfahren ersichtlich ist, ist das Protein ARAP11 für die Durchführung der Verfahren bestens geeignet. Gegenstand der vorliegenden Erfindung ist deshalb ferner das die folgende Aminosäuresequenz aufweisende ARAP11 
Met Val Val Leu Arg Ser Ser Leu Glu Leu His Asn His Ser Ala Ala
1               5                   10                  15

Ser Ala Thr Gly Ser Leu Asp Leu Ser Ser Asp Phe Leu Ser Leu Glu
            20                  25                  30

His Ile Gly Arg Arg Arg Leu Arg Ser Ala Gly Ala Ala Gln Lys Lys
        35                  40                  45

Pro Ala Ala Thr Thr Ala Lys Ala Gly Asp Gly Ser Ser Val Lys Glu
    50                  55                  60

Val Glu Thr Tyr His Arg Thr Arg Ala Leu Arg Ser Leu Arg Lys Asp
65                  70                  75                  80

Ala Gln Asn Ser Ser Asp Ser Ser Phe Glu Lys Asn Val Glu Ile Thr
                85                  90                  95

Glu Gln Leu Ala Asn Gly Arg His Phe Thr Arg Gln Leu Ala Arg Gln
            100                 105                 110

Gln Ala Asp Lys Lys Lys Glu Glu His Arg Glu Asp Lys Val Ile Pro
        115                 120                 125

Val Thr Arg Ser Leu Arg Ala Arg Asn Ile Val Gln Ser Thr Glu His
    130                 135                 140

Leu His Glu Asp Asn Gly Asp Val Glu Val Arg Arg Ser Cys Arg Ile
145                 150                 155                 160

Arg Ser Arg Tyr Ser Gly Val Asn Gln Ser Met Leu Phe Asp Lys Leu
                165                 170                 175

Ile Thr Asn Thr Ala Glu Ala Val Leu Gln Lys Met Asp Asp Met Lys
            180                 185                 190
 
Lys Met Arg Arg Gln Arg Met Arg Glu Leu Glu Asp Leu Gly Val Phe
        195                 200                 205

Asn Glu Thr Glu Glu Ser Asn Leu Asn Met Tyr Thr Arg Gly Lys Gln
    210                 215                 220

Lys Asp Ile Gln Arg Thr Asp Glu Glu Thr Thr Asp Asn Gln Glu Gly
225                 230                 235                 240

Ser Val Glu Ser Ser Glu Glu Gly Glu Asp Gln Glu His Glu Asp Asp
                245                 250                 255

Gly Glu Asp Glu Asp Asp Glu Asp Asp Asp Asp Asp Asp Asp Asp Asp
            260                 265                 270

Asp Asp Asp Asp Asp Glu Asp Asp Glu Asp Glu Glu Asp Gly Glu Glu
        275                 280                 285

Glu Asn Gln Lys Arg Tyr Tyr Leu Arg Gln Arg Lys Ala Thr Val Tyr
    290                 295                 300

Tyr Gln Ala Pro Leu Glu Lys Pro Arg His Gln Arg Lys Pro Asn Ile
305                 310                 315                 320

Phe Tyr Ser Gly Pro Ala Ser Pro Ala Arg Pro Arg Tyr Arg Leu Ser
                325                 330                 335

Ser Ala Gly Pro Arg Ser Pro Tyr Cys Lys Arg Met Asn Arg Arg Arg
            340                 345                 350

His Ala Ile His Ser Ser Asp Ser Thr Ser Ser Ser Ser Glu Asp
        355                 360                 365

Glu Gln His Phe Glu Arg Arg Arg Lys Arg Ser Arg Asn Arg Ala Ile
        370                 375                 380

Asn Arg Cys Leu Pro Leu Asn Phe Arg Lys Asp Glu Leu Lys Gly Ile
385 390 395 400

Tyr Lys Asp Arg Met Lys Ile Gly Ala Ser Leu Ala Asp Val Asp Pro
405 410 415

Met Gln Leu Asp Ser Ser Val Arg Phe Asp Ser Val Gly Gly Leu Ser
                420                 425                 430

Asn His Ile Ala Ala Leu Lys Glu Met Val Val Phe Pro Leu Leu Tyr
            435                 440                 445

Pro Glu Val Phe Glu Lys Phe Lys Ile Gln Pro Pro Arg Gly Cys Leu
        450                 455                 460

Phe Tyr Gly Pro Pro Gly Thr Gly Lys Thr Leu Val Ala Arg Ala Leu
465 470 475 480

Ala Asn Glu Cys Ser Gln Gly Asp Lys Arg Val Ala Phe Phe Met Arg
485 490 495

Lys Gly Ala Asp Cys Leu Ser Lys Trp Val Gly Glu Ser Glu Arg Gln
                500                 505                 510

Leu Arg Leu Leu Phe Asp Gln Ala Tyr Gln Met Arg Pro Ser Ile Ile
            515                 520                 525

Phe Phe Asp Glu Ile Asp Gly Leu Ala Pro Val Arg Ser Ser Arg Gln
        530                 535                 540
 
Asp Gln Ile His Ser Ser Ile Val Ser Thr Leu Leu Ala Leu Met Asp
545                 550                 555                 560

Gly Leu Asp Ser Arg Gly Glu Ile Val Val Ile Gly Ala Thr Asn Arg
                565                 570                 575

Leu Asp Ser Ile Asp Pro Ala Leu Arg Arg Pro Gly Arg Phe Asp Arg
            580                 585                 590

Glu Phe Leu Phe Ser Leu Pro Asp Lys Glu Ala Arg Lys Glu Ile Leu
        595                 600                 605

Lys Ile His Thr Arg Asp Trp Asn Pro Lys Pro Leu Asp Thr Phe Leu
    610                 615                 620

Glu Glu Leu Ala Glu Asn Cys Val Gly Tyr Cys Gly Ala Asp Ile Lys
625                 630                 635                 640

Ser Ile Cys Ala Glu Ala Ala Leu Cys Ala Leu Arg Arg Arg Tyr Pro
                645                 650                 655

Gln Ile Tyr Thr Thr Ser Glu Lys Leu Gln Leu Asp Leu Ser Ser Ile
            660                 665                 670

Asn Ile Ser Ala Lys Asp Phe Glu Val Ala Met Gln Lys Met Ile Pro
        675                 680                 685

Ala Ser Gln Arg Ala Val Thr Ser Pro Gly Gln Ala Leu Ser Thr Val
    690                 695                 700

Val Lys Pro Leu Leu Gln Asn Thr Val Asp Lys Ile Leu Glu Ala Leu
705                 710                 715                 720

Gln Arg Val Phe Pro His Ala Glu Phe Arg Thr Asn Lys Thr Leu Asp
                725                 730                 735

Ser Asp Ile Ser Cys Pro Leu Leu Glu Ser Asp Leu Ala Tyr Ser Asp
            740                 745                 750

Asp Asp Val Pro Ser Val Tyr Glu Asn Gly Leu Ser Gln Lys Ser Ser
        755                 760                 765

His Lys Ala Lys Asp Asn Phe Asn Phe Leu His Leu Asn Arg Asn Ala
    770                 775                 780

Cys Tyr Gln Pro Met Ser Phe Arg Pro Arg Ile Leu Ile Val Gly Glu
785                 790                 795                 800

Pro Gly Phe Gly Gln Gly Ser His Leu Ala Pro Ala Val Ile His Ala
                805                 810                 815

Leu Glu Lys Phe Thr Val Tyr Thr Leu Asp Ile Pro Val Leu Phe Gly
            820                 825                 830

Val Ser Thr Thr Ser Pro Glu Glu Thr Cys Ala Gln Val Ile Arg Glu
        835                 840                 845

Ala Lys Arg Thr Ala Pro Ser Ile Val Tyr Val Pro His Ile His Val
    850                 855                 860

Trp Trp Glu Ile Val Gly Pro Thr Leu Lys Ala Thr Phe Thr Thr Leu
865                 870                 875                 880

Leu Gln Asn Ile Pro Ser Phe Ala Pro Val Leu Leu Leu Ala Thr Ser
                885                 890                 895
 
Asp Lys Pro His Ser Ala Leu Pro Glu Glu Val Gln Glu Leu Phe Ile
                900                 905                 910

Arg Asp Tyr Gly Glu Ile Phe Asn Val Gln Leu Pro Asp Lys Glu Glu
            915                 920                 925

Arg Thr Lys Phe Phe Glu Asp Leu Ile Leu Lys Gln Ala Ala Lys Pro
        930                 935                 940

Pro Ile Ser Lys Lys Lys Ala Val Leu Gln Ala Leu Glu Val Leu Pro
945 950 955 960

Val Ala Pro Pro Pro Glu Pro Arg Ser Leu Thr Ala Glu Glu Val Lys
965 970 975

Arg Leu Glu Glu Gln Glu Glu Asp Thr Phe Arg Glu Leu Arg Ile Phe
                980                 985                 990

Leu Arg Asn Val Thr His Arg Leu Ala Ile Asp Lys Arg Phe Arg Val
            995                 1000                1005

Phe Thr Lys Pro Val Asp Pro Asp Glu Val Pro Asp Tyr Val Thr Val
        1010                1015                1020

Ile Lys Gln Pro Met Asp Leu Ser Ser Val Ile Ser Lys Ile Asp Leu
1025 1030 1035 1040

His Lys Tyr Leu Thr Val Lys Asp Tyr Leu Arg Asp Ile Asp Leu Ile
1045 1050 1055

Cys Ser Asn Ala Leu Glu Tyr Asn Pro Asp Arg Asp Pro Gly Asp Arg
                1060                1065                1070

Leu Ile Arg His Arg Ala Cys Ala Leu Arg Asp Thr Ala Tyr Ala Ile
            1075                1080                1085

Ile Lys Glu Glu Leu Asp Glu Asp Phe Glu Gln Leu Cys Glu Glu Ile
        1090                1095                1100

Gln Glu Ser Arg Lys Lys Arg Gly Cys Ser Ser Ser Lys Tyr Ala Pro
1105 1110 1115 1120

Ser Tyr Tyr His Val Met Pro Lys Gln Asn Ser Thr Leu Val Gly Asp
1125 1130 1135

Lys Arg Ser Asp Pro Glu Gln Asn Glu Lys Leu Lys Thr Pro Ser Thr
                1140                1145                1150

Pro Val Ala Cys Ser Thr Pro Ala Gln Leu Lys Arg Lys Ile Arg Lys
            1155                1160                1165

Lys Ser Asn Trp Tyr Leu Gly Thr Ile Lys Lys Arg Arg Lys Ile Ser
        1170                1175                1180

Gln Ala Lys Asp Asp Ser Gln Asn Ala Ile Asp His Lys Ile Glu Ser
1185 1190 1195 1200

Asp Thr Glu Glu Thr Gln Asp Thr Ser Val Asp His Asn Glu Thr Gly
1205 1210 1215

Asn Thr Gly Glu Ser Ser Val Glu Glu Asn Glu Lys Gln Gln Asn Ala
                1220                1225                1230

Ser Glu Ser Lys Leu Glu Leu Arg Asn Asn Ser Asn Thr Cys Asn Ile
            1235                1240                1245
 
Glu Asn Glu Leu Glu Asp Ser Arg Lys Thr Thr Ala Cys Thr Glu Leu
        1250                1255                1260

Arg Asp Lys Ile Ala Cys Asn Gly Asp Ala Ser Ser Ser Gln Ile Ile
1265 1270 1275 1280

His Ile Ser Asp Glu Asn Glu Gly Lys Glu Met Cys Val Leu Arg Met
1285 1290 1295

Thr Arg Ala Arg Arg Ser Gln Val Glu Gln Gln Gln Leu Ile Thr Val
                1300                1305                1310

Glu Lys Ala Leu Ala Ile Leu Ser Gln Pro Thr Pro Ser Leu Val Val
            1315                1320                1325

Asp His Glu Arg Leu Lys Asn Leu Leu Lys Thr Val Val Lys Lys Ser
        1330                1335                1340

Gln Asn Tyr Asn Ile Phe Gln Leu Glu Asn Leu Tyr Ala Val Ile Ser
1345 1350 1355 1360

Gln Cys Ile Tyr Arg His Arg Lys Asp His Asp Lys Thr Ser Leu Ile
1365 1370 1375

Gln Lys Met Glu Gln Glu Val Glu Asn Phe Ser Cys Ser Arg
1380 1385 1390
 oder das die Aminosäuren 813 bis 1390 dieses Proteins aufweisende ARAP11-Fragment. Gegenstand der vorliegenden Anmeldung ist ferner ein für ARAP11 oder dessen Fragment, insbesondere das die Aminosäuren 813 bis 1390 aufweisende Fragment, kodierende DNA sowie eine daran hybridisierende DNA. Der Ausdruck "hybridisierende DNA" weist auf eine DNA hin, die unter üblichen Bedingungen, insbesondere bei 20 °C unter dem Schmelzpunkt der DNA, mit der kodierenden DNA hybridisiert.

Die Erfindung wird unter Bezugnahme auf die folgenden Abbildungen näher erläutert, wobei
Abbildung 1 eine schematische Darstellung des Androgenrezeptors mit Kennzeichnung der von den Aminosäuren 325 bis 919 reichenden Androgenrezeptor-Domäne (AR 2) ist, die zur Interaktion mit ARAP11 in Abwesenheit von Androgen fähig ist;
Abbildung 2 die Gewebsverteilung von ARAP11 darstellt;
Abbildung 3 die Co-Aktivierung des Androgenrezeptor-Signals in SH-SY5Y-Zellen, und
Abbildung 4 die Expression von ARAP11 und β-Actin in Hoden von Ratten zeigt.

Das folgende Beispiel illustriert die Erfindung näher, ohne sie jedoch darauf zu beschränken.

### Beispiel 1: Co-Aktivierung des Androgenrezeptor-Signals durch ARAP11

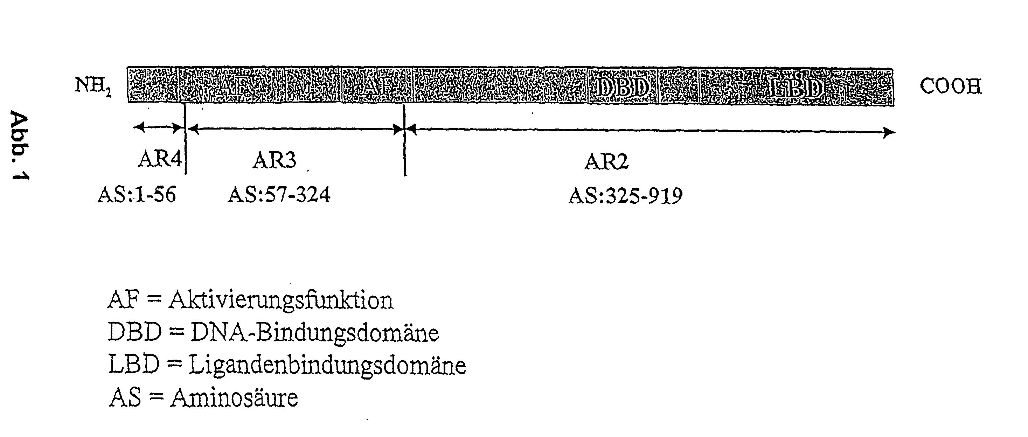

Unter Verwendung einer cDNA-Bibliothek aus fetalem Gehirn (Clontech MATCHMAKER) und eines humanen AR-Fragments, das für die Aminosäuren 325 bis 919 kodiert, als Sonde (Abbildung 1) wurde ein Screening mittels eines üblichen Zwei-Hybrid-Hefe-Systems in Abwesenheit von Androgen durchgeführt. In Übereinstimmung mit den Anweisungen des Herstellers (Clontech) betrug die Zahl der gescreenten Klone 6x10⁷. Die Zahl der unabhängigen Klone betrug laut Angaben des Herstellers 3,5x10⁶. Davon wurden 350 positive Klone ausgewählt und mit einem β-Galaktosidase-Assay getestet, wobei 240 als lacZ-positive Klone bestätigt wurden. Die Inserts dieser Klone wurden durch PCR amplifiziert. Mittels Restriktionsfragmentanalysen und Sequenzierung wurden mindestens 17 verschiedene Klone identifiziert. Einer davon war ein Klon mit einem 1169 bp umfassenden Insert (3243 bp - 4412 bp), das für einen Teil des ORF (Open reading frame) kodiert. Diese Sequenz enthält auch fast den vollständigen Teil des bereits in Pro2000 (Genbank-Zugangsnummer XM005253) beschriebenen ORFs.

Mit Hilfe eines üblichen PCR-Verfahrens wurde dann in voller Länge die codierende ARAP11-cDNA kloniert, die für ein aus 1390 Aminosäuren bestehendes Protein (SEQ ID No. 2) kodiert und erheblich über die bisher bekannte Pro 2000 Sequenz hinausgeht, welches ein Protein von 362 Aminosäure beschreibt. Zusammen mit dem 5' und 3' nicht translatierten Bereichen hat die hier beschriebene Sequenz eine Länge von 4412 bp (SEQ ID No.1).

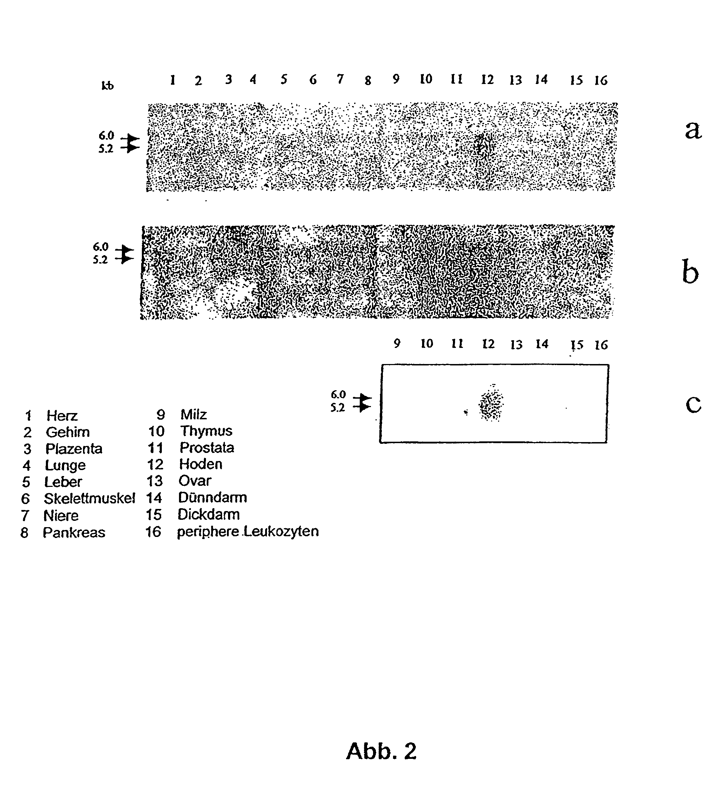

Abbildung 2 zeigt die Gewebsverteilung von ARAP11, die in an sich bekannter Weise mittels Northern-Blot-Analyse ermittelt wurde. Aus verschiedenen humanen Geweben isolierte Poly-A+-RNA (2 µg) wurde mit einem Formaldehyd-haltigem Agarosegel aufgetrennt, auf eine Nylonmembran geblottet und mit einem markierten ARAP11-cDNA Fragment hybridisiert. Für das in Abb. 2a und 2b beschriebene Experiment wurde ein Fragment von 3111 bis 4217 bp eingesetzt, für das in Abb. 2c ein Fragment von 2065-2476 bp der cDNA-Sequenz von ARAP11. Nach dem Waschen wurde die Membran auf einen Film gelegt und nach Belichtung für 24 Stunden (Abb. 2a und 2c) oder 8 Tagen (Abb. 2b) entwickelt. Wie Abbildung 2 entnommen werden kann, wurde in Hoden eine sehr starke, in Leber, Herz, Thymus und Prostata dagegen eine schwächere Expression von ARAP11 nachgewiesen. Dabei wurden zwei Transkripte (6,0 kb und 5,2kb) entdeckt.

Mit der Sonde aus dem 411 bp bestehenden Fragment von 2065 bis 2476 bp der cDNA-Sequenz von ARAP11 wurden wieder zwei gleich große Transkripte in Hoden nachgewiesen, so daß davon ausgegangen werden kann, daß es sich bei den nachgewiesenen Transkripten, um die identischen Transkripte handelt, welche unter 2a und 2b mit der Sonde von 3111-4217 nachgewiesen wurden. Dies stellt ein Beleg dafür dar, daß die in der Genbank unter XM005253 hinterlegte Sequenz von Pro 2000 unvollständig und im 5'-Bereich um 2480 bp länger ist.

Die mittels PCR erzeugte ARAP11 cDNA die für das ARAP 11-Fragment von den Aminosäuren 813 bis 1390 kodiert, wurde in üblicher Weise in den Vektor CMX kloniert und mit pSG5-AR und MMTV-Luciferase in SH-SY5Y-Zellen ebenfalls in üblicher Weise transfiziert.

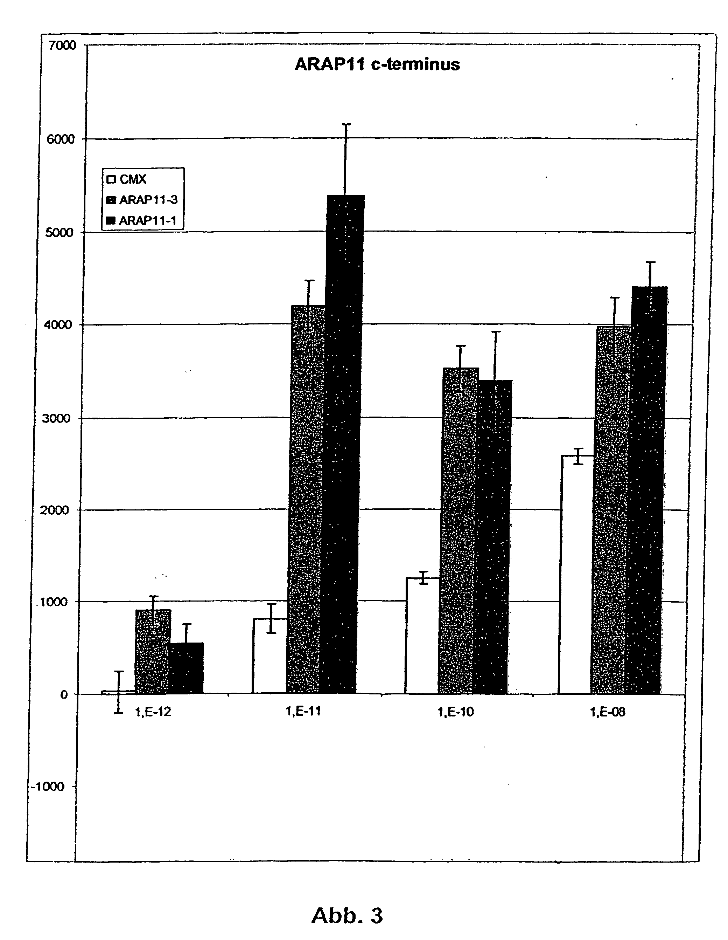

Wie Abbildung 3 entnommen werden kann, führte die transiente Transfektion von ARAP11-cDNA in SH-SY5Y-Zellen zu einer starken Co-Aktivierung der AR-Signaltätigkeit, insbesondere bei niedrigen Androgenkonzentrationen von 10⁻¹⁰-10⁻¹² M. Dazu wurden in eine Zellkulturschale mit Vertiefungen pro Vertiefung 3x10⁵- Zellen mit 1 µg Co-Aktivator- (ARAP11-3 bzw.ARAP11-1, jeweils kodierend für Aminosäuren 813 bis 1390 von ARAP11) in CMX bzw 1µg CMX als Kontrollplasmid, 1,5 µg MMTV-Luciferase- und 0,75 µg pSG5AR-Plasmid transfiziert und nach 24 Stunden mit Dihydroxytestosteron (DHT) als Androgen in den angegebenen Konzentrationen behandelt. Die transfizierten Zellen wurden nach weiteren 24 Stunden geerntet und die Aktivität des Reportergens Luziferase gemessen. Zusätzlich wurde zur Normierung die Gesamtzellproteinmenge bestimmt. Pro Transfektionsansatz und Substanzkonzentration wurde ein Experiment und jeweils vier Messungen durchgeführt. Die Fehlerabweichung wurde als SD angegeben. Bei allen Signalen wurden die Werte der entsprechenden Kontrollen ohne DHT subtrahiert. Die Aktivität ist in relativen Einheiten angegeben.

### Beispiel 2: Bestimmung von ARAP11 in Hoden von Ratten

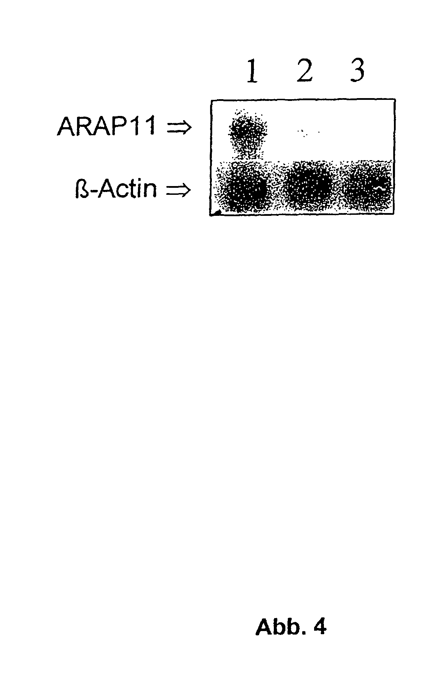

Abbildung 4 zeigt die Expression von ARAP11 und β-Actin in Hoden von Ratten. Aus Geweben von Rattenhoden wurden Poly-A+RNAs (4µg) isoliert, mit einem Formaldeydhaltigem Agarosegel aufgetrennt, auf eine Nylonmembran übertragen und entweder mit einem markierten ARAP11-cDNA Fragment (2226 - 4228 bp) oder einer markierten β-Actin cDNA (Ratte) hybridisiert. Nach dem Waschen wurde die Membran auf einen Film gelegt und nach Belichtung für 5 Tagen entwickelt. Im Hodengewebe der Ratte konnte ein RNA Transcript (6.0 kb) entdeckt werden. In Spalte 1 wurde die isolierte RNA von 3 Wochen alten, in Spalte 2 von 6 Wochen alten und in Spalte 3 von 2 Jahre alten Tieren aufgetragen. Man kann eine deutliche altersbedingte Abhängigkeit der Expression der ARAP11 Gens erkennen, wobei die Expression des β-Actin Gens keine Veränderung aufweist. 6 Wochen nach der Geburt ist die Expression von ARAP11 deutlich reduziert (mehr als 50%) und in alten Tieren (2 Jahre) ist nur noch sehr geringe Expression des ARAP11 Gens erkennbar. Ein ähnliches Verhalten einer Veränderung der Genexpression des Co-modulators ARAP11 ist bei Krankheitsbildern zu erwarten.

## Patentansprüche

1. Verfahren zur Testung des hormonellen Effekts, insbesondere des androgenen oder antiandrogenen Effekts, von Substanzen, bei dem
a) Zellen, die mit zwei Vektoren transfektiert sind, wobei der eine Vektor DNA, die für ein nukleäres Rezeptor-Protein oder ein Fragment davon kodiert, und der andere Vektor DNA enthält, die für einen Co-Modulator oder ein Fragment davon kodiert, der Substanz ausgesetzt werden und
b) die Transkriptionsaktivität, die der nukleäre Rezeptor oder dessen Fragment in Anwesenheit des Co-Modulators oder dessen Fragment auslöst, und/oder der Einfluss der Substanz auf die Interaktion zwischen dem Rezeptor oder dessen Fragment und dem Co-Modulator oder dessen Fragment durch Protein-Protein-Interaktion oder Protein-Protein-DNA-Interaktion gemessen wird,
wobei der Co-Modulator ARAP11 ist, das die folgende Aminosäuresequenz umfaßt:

Met Val Val Leu Arg Ser Ser Leu Glu Leu His Asn His Ser Ala Ala
1               5                   10                  15

Ser Ala Thr Gly Ser Leu Asp Leu Ser Ser Asp Phe Leu Ser Leu Glu
            20                  25                  30

His Ile Gly Arg Arg Arg Leu Arg Ser Ala Gly Ala Ala Gln Lys Lys
        35                  40                  45

Pro Ala Ala Thr Thr Ala Lys Ala Gly Asp Gly Ser Ser Val Lys Glu
    50                  55                  60

Val Glu Thr Tyr His Arg Thr Arg Ala Leu Arg Ser Leu Arg Lys Asp
65                  70                  75                  80

Ala Gln Asn Ser Ser Asp Ser Ser Phe Glu Lys Asn Val Glu Ile Thr
                85                  90                  95

Glu Gln Leu Ala Asn Gly Arg His Phe Thr Arg Gln Leu Ala Arg Gln
            100                 105                 110

Gln Ala Asp Lys Lys Lys Glu Glu His Arg Glu Asp Lys Val Ile Pro
        115                 120                 125

Val Thr Arg Ser Leu Arg Ala Arg Asn Ile Val Gln Ser Thr Glu His
    130                 135                 140

Leu His Glu Asp Asn Gly Asp Val Glu Val Arg Arg Ser Cys Arg Ile
145                 150                 155                 160

Arg Ser Arg Tyr Ser Gly Val Asn Gln Ser Met Leu Phe Asp Lys Leu
                165                 170                 175

Ile Thr Asn Thr Ala Glu Ala Val Leu Gln Lys Met Asp Asp Met Lys
            180                 185                 190

Lys Met Arg Arg Gln Arg Met Arg Glu Leu Glu Asp Leu Gly Val Phe
        195                 200                 205

Asn Glu Thr Glu Glu Ser Asn Leu Asn Met Tyr Thr Arg Gly Lys Gln
    210                 215                 220

Lys Asp Ile Gln Arg Thr Asp Glu Glu Thr Thr Asp Asn Gln Glu Gly
225                 230                 235                 240

Ser Val Glu Ser Ser Glu Glu Gly Glu Asp Gln Glu His Glu Asp Asp
                245                 250                 255

Gly Glu Asp Glu Asp Asp Glu Asp Asp Asp Asp Asp Asp Asp Asp Asp
            260                 265                 270

Asp Asp Asp Asp Asp Glu Asp Asp Glu Asp Glu Glu Asp Gly Glu Glu
        275                 280                 285

Glu Asn Gln Lys Arg Tyr Tyr Leu Arg Gln Arg Lys Ala Thr Val Tyr
    290                 295                 300

Tyr Gln Ala Pro Leu Glu Lys Pro Arg His Gln Arg Lys Pro Asn Ile
305                 310                 315                 320

Phe Tyr Ser Gly Pro Ala Ser Pro Ala Arg Pro Arg Tyr Arg Leu Ser
                325                 330                 335

Ser Ala Gly Pro Arg Ser Pro Tyr Cys Lys Arg Met Asn Arg Arg Arg
            340                 345                 350

His Ala Ile His Ser Ser Asp Ser Thr Ser Ser Ser Ser Ser Glu Asp
        355                 360                 365

Glu Gln His Phe Glu Arg Arg Arg Lys Arg Ser Arg Asn Arg Ala Ile
    370                 375                 380

Asn Arg Cys Leu Pro Leu Asn Phe Arg Lys Asp Glu Leu Lys Gly Ile
385                 390                 395                 400

Tyr Lys Asp Arg Met Lys Ile Gly Ala Ser Leu Ala Asp Val Asp Pro
                405                 410                 415

Met Gln Leu Asp Ser Ser Val Arg Phe Asp Ser Val Gly Gly Leu Ser
            420                 425                 430

Asn His Ile Ala Ala Leu Lys Glu Met Val Val Phe Pro Leu Leu Tyr
        435                 440                 445

Pro Glu Val Phe Glu Lys Phe Lys Ile Gln Pro Pro Arg Gly Cys Leu
    450                 455                 460

Phe Tyr Gly Pro Pro Gly Thr Gly Lys Thr Leu Val Ala Arg Ala Leu
465                 470                 475                 480

Ala Asn Glu Cys Ser Gln Gly Asp Lys Arg Val Ala Phe Phe Met Arg
                485                 490                 495

Lys Gly Ala Asp Cys Leu Ser Lys Trp Val Gly Glu Ser Glu Arg Gln
            500                 505                 510

Leu Arg Leu Leu Phe Asp Gln Ala Tyr Gln Met Arg Pro Ser Ile Ile
        515                 520                 525

Phe Phe Asp Glu Ile Asp Gly Leu Ala Pro Val Arg Ser Ser Arg Gln
    530                 535                 540

Asp Gln Ile His Ser Ser Ile Val Ser Thr Leu Leu Ala Leu Met Asp
545                 550                 555                 560

Gly Leu Asp Ser Arg Gly Glu Ile Val Val Ile Gly Ala Thr Asn Arg
                565                 570                 575

Leu Asp Ser Ile Asp Pro Ala Leu Arg Arg Pro Gly Arg Phe Asp Arg
            580                 585                 590

Glu Phe Leu Phe Ser Leu Pro Asp Lys Glu Ala Arg Lys Glu Ile Leu
        595                 600                 605

Lys Ile His Thr Arg Asp Trp Asn Pro Lys Pro Leu Asp Thr Phe Leu
    610                 615                 620

Glu Glu Leu Ala Glu Asn Cys Val Gly Tyr Cys Gly Ala Asp Ile Lys
625                 630                 635                 640

Ser Ile Cys Ala Glu Ala Ala Leu Cys Ala Leu Arg Arg Arg Tyr Pro
                645                 650                 655

Gln Ile Tyr Thr Thr Ser Glu Lys Leu Gln Leu Asp Leu Ser Ser Ile
            660                 665                 670

Asn Ile Ser Ala Lys Asp Phe Glu Val Ala Met Gln Lys Met Ile Pro
        675                 680                 685

Ala Ser Gln Arg Ala Val Thr Ser Pro Gly Gln Ala Leu Ser Thr Val
    690                 695                 700

Val Lys Pro Leu Leu Gln Asn Thr Val Asp Lys Ile Leu Glu Ala Leu
705                 710                 715                 720

Gln Arg Val Phe Pro His Ala Glu Phe Arg Thr Asn Lys Thr Leu Asp
                725                 730                 735

Ser Asp Ile Ser Cys Pro Leu Leu Glu Ser Asp Leu Ala Tyr Ser Asp
            740                 745                 750

Asp Asp Val Pro Ser Val Tyr Glu Asn Gly Leu Ser Gln Lys Ser Ser
        755                 760                 765

His Lys Ala Lys Asp Asn Phe Asn Phe Leu His Leu Asn Arg Asn Ala
    770                 775                 780

Cys Tyr Gln Pro Met Ser Phe Arg Pro Arg Ile Leu Ile Val Gly Glu
785                 790                 795                 800

Pro Gly Phe Gly Gln Gly Ser His Leu Ala Pro Ala Val Ile His Ala
                805                 810                 815

Leu Glu Lys Phe Thr Val Tyr Thr Leu Asp Ile Pro Val Leu Phe Gly
            820                 825                 830

Val Ser Thr Thr Ser Pro Glu Glu Thr Cys Ala Gln Val Ile Arg Glu
        835                 840                 845

Ala Lys Arg Thr Ala Pro Ser Ile Val Tyr Val Pro His Ile His Val
    850                 855                 860

Trp Trp Glu Ile Val Gly Pro Thr Leu Lys Ala Thr Phe Thr Thr Leu
865                 870                 875                 880

Leu Gln Asn Ile Pro Ser Phe Ala Pro Val Leu Leu Leu Ala Thr Ser
                885                 890                 895

Asp Lys Pro His Ser Ala Leu Pro Glu Glu Val Gln Glu Leu Phe Ile
            900                 905                 910

Arg Asp Tyr Gly Glu Ile Phe Asn Val Gln Leu Pro Asp Lys Glu Glu
        915                 920                 925

Arg Thr Lys Phe Phe Glu Asp Leu Ile Leu Lys Gln Ala Ala Lys Pro
    930                 935                 940

Pro Ile Ser Lys Lys Lys Ala Val Leu Gln Ala Leu Glu Val Leu Pro
945                 950                 955                 960

Val Ala Pro Pro Pro Glu Pro Arg Ser Leu Thr Ala Glu Glu Val Lys
                965                 970                 975

Arg Leu Glu Glu Gln Glu Glu Asp Thr Phe Arg Glu Leu Arg Ile Phe
            980                 985                 990

Leu Arg Asn Val Thr His Arg Leu Ala Ile Asp Lys Arg Phe Arg Val
        995                 1000                1005

Phe Thr Lys Pro Val Asp Pro Asp Glu Val Pro Asp Tyr Val Thr Val
    1010                1015                1020

Ile Lys Gln Pro Met Asp Leu Ser Ser Val Ile Ser Lys Ile Asp Leu
1025                1030                1035                1040

His Lys Tyr Leu Thr Val Lys Asp Tyr Leu Arg Asp Ile Asp Leu Ile
                1045                1050                1055

Cys Ser Asn Ala Leu Glu Tyr Asn Pro Asp Arg Asp Pro Gly Asp Arg
            1060                1065                1070

Leu Ile Arg His Arg Ala Cys Ala Leu Arg Asp Thr Ala Tyr Ala Ile
        1075                1080                1085

Ile Lys Glu Glu Leu Asp Glu Asp Phe Glu Gln Leu Cys Glu Glu Ile
    1090                1095                1100

Gln Glu Ser Arg Lys Lys Arg Gly Cys Ser Ser Ser Lys Tyr Ala Pro
1105                1110                1115                1120

Ser Tyr Tyr His Val Met Pro Lys Gln Asn Ser Thr Leu Val Gly Asp
                1125                1130                1135

Lys Arg Ser Asp Pro Glu Gln Asn Glu Lys Leu Lys Thr Pro Ser Thr
            1140                1145                1150

Pro Val Ala Cys Ser Thr Pro Ala Gln Leu Lys Arg Lys Ile Arg Lys
        1155                1160                1165

Lys Ser Asn Trp Tyr Leu Gly Thr Ile Lys Lys Arg Arg Lys Ile Ser
    1170                1175                1180

Gln Ala Lys Asp Asp Ser Gln Asn Ala Ile Asp His Lys Ile Glu Ser
1185                1190                1195                1200

Asp Thr Glu Glu Thr Gln Asp Thr Ser Val Asp His Asn Glu Thr Gly
                1205                1210                1215

Asn Thr Gly Glu Ser Ser Val Glu Glu Asn Glu Lys Gln Gln Asn Ala
            1220                1225                1230

Ser Glu Ser Lys Leu Glu Leu Arg Asn Asn Ser Asn Thr Cys Asn Ile
        1235                1240                1245

Glu Asn Glu Leu Glu Asp Ser Arg Lys Thr Thr Ala Cys Thr Glu Leu
    1250                1255                1260

Arg Asp Lys Ile Ala Cys Asn Gly Asp Ala Ser Ser Ser Gln Ile Ile
1265                1270                1275                1280

His Ile Ser Asp Glu Asn Glu Gly Lys Glu Met Cys Val Leu Arg Met
                1285                1290                1295

Thr Arg Ala Arg Arg Ser Gln Val Glu Gln Gln Gln Leu Ile Thr Val
            1300                1305                1310

Glu Lys Ala Leu Ala Ile Leu Ser Gln Pro Thr Pro Ser Leu Val Val
        1315                1320                1325

Asp His Glu Arg Leu Lys Asn Leu Leu Lys Thr Val Val Lys Lys Ser
    1330                1335                1340

Gln Asn Tyr Asn Ile Phe Gln Leu Glu Asn Leu Tyr Ala Val Ile Ser
1345                1350                1355                1360

Gln Cys Ile Tyr Arg His Arg Lys Asp His Asp Lys Thr Ser Leu Ile
                1365                1370                1375

Gln Lys Met Glu Gln Glu Val Glu Asn Phe Ser Cys Ser Arg
            1380                1385                1390

2. Verfahren nach Anspruch 1, wobei das Fragment des Co-Modulators die Aminosäuren 813 bis 1390 von ARAP11 aufweist.

3. Verfahren nach einem der vorhergehenden Ansprüche, wobei der nukleäre Rezeptor ausgewählt ist unter Androgenrezeptor, Estrogenrezeptor α, Estrogenrezeptor β, Progesteronrezeptor A, Progesteronrezeptor B, Glukokortikoidrezeptor, Mineralokortikoidrezeptor, Schilddrüsenhormonrezeptor, Vitamin-D-Rezeptor, Peroxisomproliferator-aktivierter Rezeptor, Retinsäurerezeptor, Retinoid-X-Rezeptor und Orphan-Rezeptoren.

4. Verfahren nach einem der vorhergehenden Ansprüche, wobei die Zellen etablierte Zelllinien und/oder eukaryotische Zellen sind.

5. Verfahren nach Anspruch 4, wobei die eukaryotischen Zellen unter Prostatazellen, Nervenzellen, Gliazellen, Fibroblasten, Blutzellen, Osteoblasten, Osteoklasten, Hepatozyten, Epithelzellen oder Muskelzellen ausgewählt sind.

6. Verfahren nach einem der vorhergehenden Ansprüche, wobei der Vektor ein eukaryotischer Expressionsvektor ist.

7. Verfahren zur Bestimmung von Störungen im Co-Modulationsmechanismus zwischen Androgenrezeptoren und ARAP11 wobei die Konzentrationen von ARAP11 oder einem Fragment davon und/oder Androgenrezeptor oder einem Fragment davon gemessen werden.

8. Verfahren nach Anspruch 7, wobei die Konzentrationsmessung durch Radioimmunassay, ELISA-Tests, Immunfärbung, RT-PCR, Westem-Blot, Northem-Blot, DNA-Chip oder Protein-Chip erfolgt.

9. Protein oder Fragment davon mit Co-Modulatoreigenschaften für den Androgenrezeptor, wobei es die Aminosäuresequenz von ARAP11 gemäß Anspruch 1 aufweist.

10. Protein nach Anspruch 9, wobei das Fragment die Aminosäuren 813 bis 1390 aufweist.

11. DNA, kodierend für das Proteine nach Anspruch 9 oder 10 oder damit hybridisierende DNA.

## Claims

1. Method of testing the hormonal effect, especially the androgenic or antiandrogenic effect, of substances, wherein
a) cells transfected with two vectors, one of the vectors containing DNA that codes for a nuclear receptor protein or a fragment thereof and the other vector containing DNA that codes for a co-modulator or a fragment thereof, are exposed to the substance and
b) the transcription activity that the nuclear receptor or its fragment triggers in the presence of the comodulator or its fragment, and/or the influence of the substance on the interaction between the receptor or its fragment and the co-modulator or its fragment, is measured by protein-protein interaction or protein-protein DNA interaction,
the comodulator being ARAP11, comprising the following amino acid sequence:

Met Val Val Leu Arg Ser Ser Leu Glu Leu His Asn His Ser Ala Ala
1               5                   10                  15
Ser Ala Thr Gly Ser Leu Asp Leu Ser Ser Asp Phe Leu Ser Leu Glu
            20                  25                  30
His Ile Gly Arg Arg Arg Leu Arg Ser Ala Gly Ala Ala Gln Lys Lys
        35                  40                  45
Pro Ala Ala Thr Thr Ala Lys Ala Gly Asp Gly Ser Ser Val Lys Glu
    50                  55                  60
Val Glu Thr Tyr His Arg Thr Arg Ala Leu Arg Ser Leu Arg Lys Asp
65                  70                  75                  80
Ala Gln Asn Ser Ser Asp Ser Ser Phe Glu Lys Asn Val Glu Ile Thr
                85                  90                  95
Glu Gln Leu Ala Asn Gly Arg His Phe Thr Arg Gln Leu Ala Arg Gln
            100                 105                 110
Gln Ala Asp Lys Lys Lys Glu Glu His Arg Glu Asp Lys Val Ile Pro
        115                 120                 125

Val Thr Arg Ser Leu Arg Ala Arg Asn Ile Val Gln Ser Thr Glu His
    130                 135                 140
Leu His Glu Asp Asn Gly Asp Val Glu Val Arg Arg Ser Cys Arg Ile
145                 150                 155                 160
Arg Ser Arg Tyr Ser Gly Val Asn Gln Ser Met Leu Phe Asp Lys Leu
                165                 170                 175
Ile Thr Asn Thr Ala Glu Ala Val Leu Gln Lys Met Asp Asp Met Lys
            180                 185                 190
Lys Met Arg Arg Gln Arg Met Arg Glu Leu Gln Asp Leu Gly Val Phe
        195                 200                 205
Asn Glu Thr Glu Glu Ser Asn Leu Asn Met Tyr Thr Arg Gly Lys Gln
    210                 215                 220
Lys Asp Ile Gln Arg Thr Asp Glu Glu Thr Thr Asp Asn Gln Glu Gly
225                 230                 235                 240
Ser Val Glu Ser Ser Glu Glu Gly Glu Asp Gln Glu His Glu Asp Asp
                245                 250                 255
Gly Glu Asp Glu Asp Asp Glu Asp Asp Asp Asp Asp Asp Asp Asp Asp
            260                 265                 270
Asp Asp Asp Asp Asp Glu Asp Asp Glu Asp Glu Glu Asp Gly Glu Glu
        275                 280                 285
Glu Asn Gln Lys Arg Tyr Tyr Leu Arg Gln Arg Lys Ala Thr Val Tyr
    290                 295                 300
Tyr Gln Ala Pro Leu Glu Lys Pro Arg His Gln Arg Lys Pro Asn Ile
305                 310                 315                 320
Phe Tyr Ser Gly Pro Ala Ser Pro Ala Arg Pro Arg Tyr Arg Leu Ser
                325                 330                 335
Ser Ala Gly Pro Arg Ser Pro Tyr Cys Lys Arg Met Asn Arg Arg Arg
            340                 345                 350
His Ala Ile His Ser Ser Asp Ser Thr Ser Ser Ser Ser Ser Glu Asp
        355                 360                 365
Glu Gln His Phe Glu Arg Arg Arg Lys Arg Ser Arg Asn Arg Ala Ile
    370                 375                 380
Asn Arg Cys Leu Pro Leu Asn Phe Arg Lys Asp Glu Leu Lys Gly Ile
385                 390                 395                 400

Tyr Lys Asp Arg Met Lys Ile Gly Ala Ser Leu Ala Asp Val Asp Pro
                405                 410                 415
Met Gln Leu Asp Ser Ser Val Arg Phe Asp Ser Val Gly Gly Leu Ser
            420                 425                 430
Asn His Ile Ala Ala Leu Lys Glu Met Val Val Phe Pro Leu Leu Tyr
        435                 440                 445
Pro Glu Val Phe Glu Lys Phe Lys Ile Gln Pro Pro Arg Gly Cys Leu
    450                 455                 460
Phe Tyr Gly Pro Pro Gly Thr Gly Lys Thr Leu Val Ala Arg Ala Leu
465                 470                 475                 480
Ala Asn Glu Cys Ser Gln Gly Asp Lys Arg Val Ala Phe Phe Met Arg
                485                 490                 495
Lys Gly Ala Asp Cys Leu Ser Lys Trp Val Gly Glu Ser Glu Arg Gln
            500                 505                 510
Leu Arg Leu Leu Phe Asp Gln Ala Tyr Gln Met Arg Pro Ser Ile Ile
        515                 520                 525
Phe Phe Asp Glu Ile Asp Gly Leu Ala Pro Val Arg Ser Ser Arg Gln
    530                 535                 540
Asp Gln Ile His Ser Ser Ile Val Ser Thr Leu Leu Ala Leu Met Asp
545                 550                 555                 560
Gly Leu Asp Ser Arg Gly Glu Ile Val Val Ile Gly Ala Thr Asn Arg
                565                 570                 575
Leu Asp Ser Ile Asp Pro Ala Leu Arg Arg Pro Gly Arg Phe Asp Arg
            580                 585                 590
Glu Phe Leu Phe Ser Leu Pro Asp Lys Glu Ala Arg Lys Glu Ile Leu
        595                 600                 605
Lys Ile His Thr Arg Asp Trp Asn Pro Lys Pro Leu Asp Thr Phe Leu
    610                 615                 620
Glu Glu Leu Ala Glu Asn Cys Val Gly Tyr Cys Gly Ala Asp Ile Lys
625                 630                 635                 640
Ser Ile Cys Ala Glu Ala Ala Leu Cys Ala Leu Arg Arg Arg Tyr Pro
                645                 650                 655
Gln Ile Tyr Thr Thr Ser Glu Lys Leu Gln Leu Asp Leu Ser Ser Ile
            660                 665                 670

Asn Ile Ser Ala Lys Asp Phe Glu Val Ala Met Gln Lys Met Ile Pro
        675                 680                 685
Ala Ser Gln Arg Ala Val Thr Ser Pro Gly Gln Ala Leu Ser Thr Val
    690                 695                 700
Val Lys Pro Leu Leu Gln Asn Thr Val Asp Lys Ile Leu Glu Ala Leu
705                 710                 715                 720
Gln Arg Val Phe Pro His Ala Glu Phe Arg Thr Asn Lys Thr Leu Asp
                725                 730                 735
Ser Asp Ile Ser Cys Pro Leu Leu Glu Ser Asp Leu Ala Tyr Ser Asp
            740                 745                 750
Asp Asp Val Pro Ser Val Tyr Glu Asn Gly Leu Ser Gln Lys Ser Ser
        755                 760                 765
His Lys Ala Lys Asp Asn Phe Asn Phe Leu His Leu Asn Arg Asn Ala
    770                 775                 780
Cys Tyr Gln Pro Met Ser Phe Arg Pro Arg Ile Leu Ile Val Gly Glu
785                 790                 795                 800
Pro Gly Phe Gly Gln Gly Ser His Leu Ala Pro Ala Val Ile His Ala
                805                 810                 815
Leu Glu Lys Phe Thr Val Tyr Thr Leu Asp Ile Pro Val Leu Phe Gly
            820                 825                 830
Val Ser Thr Thr Ser Pro Glu Glu Thr Cys Ala Gln Val Ile Arg Glu
        835                 840                 845
Ala Lys Arg Thr Ala Pro Ser Ile Val Tyr Val Pro His Ile His Val
    850                 855                 860
Trp Trp Glu Ile Val Gly Pro Thr Leu Lys Ala Thr Phe Thr Thr Leu
865                 870                 875                 880
Leu Gln Asn Ile Pro Ser Phe Ala Pro Val Leu Leu Leu Ala Thr Ser
                885                 890                 895
Asp Lys Pro His Ser Ala Leu Pro Glu Glu Val Gln Glu Leu Phe Ile
            900                 905                 910
Arg Asp Tyr Gly Glu Ile Phe Asn Val Gln Leu Pro Asp Lys Glu Glu
        915                 920                 925
Arg Thr Lys Phe Phe Glu Asp Leu Ile Leu Lys Gln Ala Ala Lys Pro
    930                 935                 940

Pro Ile Ser Lys Lys Lys Ala Val Leu Gln Ala Leu Glu Val Leu Pro
945                 950                 955                 960
Val Ala Pro Pro Pro Glu Pro Arg Ser Leu Thr Ala Glu Glu Val Lys
                965                 970                 975
Arg Leu Glu Glu Gln Glu Glu Asp Thr Phe Arg Glu Leu Arg Ile Phe
            980                 985                 990
Leu Arg Asn Val Thr His Arg Leu Ala Ile Asp Lys Arg Phe Arg Val
        995                 1000                1005
Phe Thr Lys Pro Val Asp Pro Asp Glu Val Pro Asp Tyr Val Thr Val
    1010                1015                1020
Ile Lys Gln Pro Met Asp Leu Ser Ser Val Ile Ser Lys Ile Asp Leu
1025                1030                1035                1040
His Lys Tyr Leu Thr Val Lys Asp Tyr Leu Arg Asp Ile Asp Leu Ile
                1045                1050                1055
Cys Ser Asn Ala Leu Glu Tyr Asn Pro Asp Arg Asp Pro Gly Asp Arg
            1060                1065                1070
Leu Ile Arg His Arg Ala Cys Ala Leu Arg Asp Thr Ala Tyr Ala Ile
        1075                1080                1085
Ile Lys Glu Glu Leu Asp Glu Asp Phe Glu Gln Leu Cys Glu Glu Ile
    1090                1095                1100
Gln Glu Ser Arg Lys Lys Arg Gly Cys Ser Ser Ser LysTyr Ala Pro
1105                1110                1115                1120
Ser Tyr Tyr His Val Met Pro Lys Gln Asn Ser Thr Leu Val Gly Asp
                1125                1130                1135
Lys Arg Ser Asp Pro Glu Gln Asn Glu Lys Leu Lys Thr Pro Ser Thr
            1140                1145                1150
Pro Val Ala Cys Ser Thr Pro Ala Gln Leu Lys Arg Lys Ile Arg Lys
        1155                1160                1165
Lys Ser Asn Trp Tyr Leu Gly Thr Ile Lys Lys Arg Arg Lys Ile Ser
    1170                1175                1180
Gln Ala Lys Asp Asp Ser Gln Asn Ala Ile Asp His Lys Ile Glu Ser
1185                1190                1195                1200
Asp Thr Glu Glu Thr Gln Asp Thr Ser Val Asp His Asn Glu Thr Gly
                1205                1210                1215

Asn Thr Gly Glu Ser Ser Val Glu Glu Asn Glu Lys Gln Gln Asn Ala
1220 1225 1230
Ser Glu Ser Lys Leu Glu Leu Arg Asn Asn Ser Asn Thr Cys Asn Ile
1235 1240 1245
Glu Asn Glu Leu Glu Asp Ser Arg Lys Thr Thr Ala Cys Thr Glu Leu
1250 1255 1260
Arg Asp Lys Ile Ala Cys Asn Gly Asp Ala Ser Ser Ser Gln Ile Ile
1265 1270 1275 1280
His Ile Ser Asp Glu Asn Glu Gly Lys Glu Met Cys Val Leu Arg Met
1285 1290 1295
Thr Arg Ala Arg Arg Ser Gln Val Glu Gln Gln Leu Ile Thr Val
1300 1305 1310
Glu Lys Ala Leu Ala Ile Leu Ser Gln Pro Thr Pro Ser Leu Val Val
1315 1320 1325
Asp His Glu Arg Leu Lys Asn Leu Leu Lys Thr Val Val Lys Lys Ser
1330 1335 1340
Gln Asn Tyr Asn Ile Phe Gln Leu Glu Asn Leu Tyr Ala Val Ile Ser
1345 1350 1355 1360
Gln Cys Ile Tyr Arg His Arg Lys Asp His Asp Lys Thr Ser Leu Ile
1365 1370 1375
Gln Lys Met Glu Gln Glu Val Glu Asn Phe Ser Cys Ser Arg
1380 1385 1390

2. Method according to Claim 1 wherein the co-modulator fragment contains amino acids 813 to 1390 of ARAP11.

3. Method according to one of the preceding claims wherein the nuclear receptor is selected from androgen receptor, oestrogen receptor α, oestrogen receptor β, progesterone receptor A, progesterone receptor B, glucocorticoid receptor, mineralocorticoid receptor, thyroid hormone receptor, vitamin D receptor, peroxisome proliferator-activated receptor, retinoic acid receptor, retinoid X receptor and orphan receptors.

4. Method according to one of the preceding claims wherein the cells are established cell lines and/or eukaryotic cells.

5. Method according to Claim 4 wherein the eukaryotic cells are selected from prostate cells, nerve cells, glial cells, fibroblasts, blood cells, osteoblasts, osteoclasts, hepatocytes, epithelial cells and muscle cells.

6. Method according to one of the preceding claims wherein the vector is a eukaryotic expression vector.

7. Method of determining disturbances in the comodulation mechanism between androgen receptors and ARAP11, wherein the concentrations of ARAP11 or a fragment thereof and/or androgen receptor or a fragment thereof are measured.

8. Method according to Claim 7 wherein the concentrations are measured by radioimmunoassay, ELISA tests, immunostaining, RT-PCR, Western blot analysis, Northern blot analysis, DNA chip analysis or protein chip analysis.

9. Protein or protein fragment with co-modulator properties for the androgen receptor, which has the amino acid sequence of ARAP11 according to Claim 1.

10. Protein according to Claim 9 wherein the fragment contains amino acids 813 to 1390.

11. DNA coding for the protein according to Claim 9 or 10, or DNA hybridizing therewith.

## Revendications

1. Procédé pour déterminer l'effet hormonal, en particulier l'effet androgène ou anti-androgène, de substances, dans lequel
a) on expose à la substance des cellules transfectées par deux vecteurs, l'un des vecteurs contenant de l'ADN qui code pour une protéine récepteur nucléaire ou un fragment de cette dernière, et l'autre vecteur contient de l'ADN qui code pour un co-modulateur ou un fragment de ce dernier, et
b) on mesure l'activité de transcription qui déclenche le récepteur nucléaire ou son fragment en présence du co-modulateur ou de son fragment, et/ou l'influence de la substance sur l'interaction entre le récepteur ou son fragment et le co-modulateur ou son fragment sous l'effet d'une interaction protéine-protéine ou d'une interaction protéine-protéine-ADN,
le co-modulateur étant l'ARAP11 qui comprend la séquence d'acides aminés suivante :

Met Val Val Leu Arg Ser Ser Leu Glu Leu His Asn His Ser Ala Ala
1               5                   10                  15

Ser Ala Thr Gly Ser Leu Asp Leu Ser Ser Asp Phe Leu Ser Leu Glu
            20                  25                  30

His Ile Gly Arg Arg Arg Leu Arg Ser Ala Gly Ala Ala Gln Lys Lys
        35                  40                  45

Pro Ala Ala Thr Thr Ala Lys Ala Gly Asp Gly Ser Ser Val Lys Glu
    50                  55                  60

Val Glu Thr Tyr His Arg Thr Arg Ala Leu Arg Ser Leu Arg Lys Asp
65                  70                  75                  80

Ala Gln Asn Ser Ser Asp Ser Ser Phe Glu Lys Asn Val Glu Ile Thr
                85                  90                  95
Glu Gln Leu Ala Asn Gly Arg His Phe Thr Arg Gln Leu Ala Arg Gln
            100                 105                 110
Gln Ala Asp Lys Lys Lys Glu Glu His Arg Glu Asp Lys Val Ile Pro
        115                 120                 125
Val Thr Arg Ser Leu Arg Ala Arg Asn Ile Val Gln Ser Thr Glu His
    130                 135                 140
Leu His Glu Asp Asn Gly Asp Val Glu Val Arg Arg Ser Cys Arg Ile
145                 150                 155                 160
Arg Ser Arg Tyr Ser Gly Val Asn Gln Ser Met Leu Phe Asp Lys Leu
                165                 170                 175
Ile Thr Asn Thr Ala Glu Ala Val Leu Gln Lys Met Asp Asp Met Lys
            180                 185                 190
Lys Met Arg Arg Gln Arg Met Arg Glu Leu Glu Asp Leu Gly Val Phe
        195                 200                 205
Asn Glu Thr Glu Glu Ser Asn Leu Asn Met Tyr Thr Arg Gly Lys Gln
    210                 215                 220
Lys Asp Ile Gln Arg Thr Asp Glu Glu Thr Thr Asp Asn Gln Glu Gly
225                 230                 235                 240
Ser Val Glu Ser Ser Glu Glu Gly Glu Asp Gln Glu His Glu Asp Asp
                245                 250                 255
Gly Glu Asp Glu Asp Asp Glu Asp Asp Asp Asp Asp Asp Asp Asp
            260                 265                 270
Asp Asp Asp Asp Asp Glu Asp Asp Glu Asp Glu Glu Asp Gly Glu Glu
            275                 280                 285
Glu Asn Gln Lys Arg Tyr Tyr Leu Arg Gln Arg Lys Ala Thr Val Tyr
        290                 295                 300
Tyr Gln Ala Pro Leu Glu Lys Pro Arg His Gln Arg Lys Pro Asn Ile
305 310 315 320
Phe Tyr Ser Gly Pro Ala Ser Pro Ala Arg Pro Arg Tyr Arg Leu Ser
325 330 335
Ser Ala Gly Pro Arg Ser Pro Tyr Cys Lys Arg Met Asn Arg Arg Arg
                340                 345                 350
His Ala Ile His Ser Ser Asp Ser Thr Ser Ser Ser Ser Ser Glu Asp
            355                 360                 365
Glu Gln His Phe Glu Arg Arg Arg Lys Arg Ser Arg Asn Arg Ala Ile
        370                 375                 380
Asn Arg Cys Leu Pro Leu Asn Phe Arg Lys Asp Glu Leu Lys Gly Ile
385 390 395 400
Tyr Lys Asp Arg Met Lys Ile Gly Ala Ser Leu Ala Asp Val Asp Pro
405 410 415
Met Gln Leu Asp Ser Ser Val Arg Phe Asp Ser Val Gly Gly Leu Ser
                420                 425                 430

Asn His Ile Ala Ala Leu Lys Glu Met Val Val Phe Pro Leu Leu Tyr
            435                 440                 445

Pro Glu Val Phe Glu Lys Phe Lys Ile Gln Pro Pro Arg Gly Cys Leu
        450                 455                 460

Phe Tyr Gly Pro Pro Gly Thr Gly Lys Thr Leu Val Ala Arg Ala Leu
465 470 475 480

Ala Asn Glu Cys Ser Gln Gly Asp Lys Arg Val Ala Phe Phe Met Arg
485 490 495

Lys Gly Ala Asp Cys Leu Ser Lys Trp Val Gly Glu Ser Glu Arg Gln
                500                 505                 510

Leu Arg Leu Leu Phe Asp Gln Ala Tyr Gln Met Arg Pro Ser Ile Ile
            515                 520                 525

Phe Phe Asp Glu Ile Asp Gly Leu Ala Pro Val Arg Ser Ser Arg Gln
        530                 535                 540

Asp Gln Ile His Ser Ser Ile Val Ser Thr Leu Leu Ala Leu Met Asp
545 550 555 560

Gly Leu Asp Ser Arg Gly Glu Ile Val Val Ile Gly Ala Thr Asn Arg
565 570 575

Leu Asp Ser Ile Asp Pro Ala Leu Arg Arg Pro Gly Arg Phe Asp Arg
                580                 585                 590

Glu Phe Leu Phe Ser Leu Pro Asp Lys Glu Ala Arg Lys Glu Ile Leu
            595                 600                 605

Lys Ile His Thr Arg Asp Trp Asn Pro Lys Pro Leu Asp Thr Phe Leu
        610                 615                 620

Glu Glu Leu Ala Glu Asn Cys Val Gly Tyr Cys Gly Ala Asp Ile Lys
625 630 635 640

Ser Ile Cys Ala Glu Ala Ala Leu Cys Ala Leu Arg Arg Arg Tyr Pro
645 650 655

Gln Ile Tyr Thr Thr Ser Glu Lys Leu Gln Leu Asp Leu Ser Ser Ile
                660                 665                 670

Asn Ile Ser Ala Lys Asp Phe Glu Val Ala Met Gln Lys Met Ile Pro
            675                 680                 685

Ala Ser Gln Arg Ala Val Thr Ser Pro Gly Gln Ala Leu Ser Thr Val
        690                 695                 700

Val Lys Pro Leu Leu Gln Asn Thr Val Asp Lys Ile Leu Glu Ala Leu
705 710 715 720

Gln Arg Val Phe Pro His Ala Glu Phe Arg Thr Asn Lys Thr Leu Asp
725 730 735

Ser Asp Ile Ser Cys Pro Leu Leu Glu Ser Asp Leu Ala Tyr Ser Asp
                740                 745                 750

Asp Asp Val Pro Ser Val Tyr Glu Asn Gly Leu Ser Gln Lys Ser Ser
            755                 760                 765

His Lys Ala Lys Asp Asn Phe Asn Phe Leu His Leu Asn Arg Asn Ala
        770                 775                 780

Cys Tyr Gln Pro Met Ser Phe Arg Pro Arg Ile Leu Ile Val Gly Glu
785                 790                 795                 800

Pro Gly Phe Gly Gln Gly Ser His Leu Ala Pro Ala Val Ile His Ala
                805                 810                 815

Leu Glu Lys Phe Thr Val Tyr Thr Leu Asp Ile Pro Val Leu Phe Gly
            820                 825                 830

Val Ser Thr Thr Ser Pro Glu Glu Thr Cys Ala Gln Val Ile Arg Glu
        835                 840                 845

Ala Lys Arg Thr Ala Pro Ser Ile Val Tyr Val Pro His Ile His Val
    850                 855                 860

Trp Trp Glu Ile Val Gly Pro Thr Leu Lys Ala Thr Phe Thr Thr Leu
865                 870                 875                 880

Leu Gln Asn Ile Pro Ser Phe Ala Pro Val Leu Leu Leu Ala Thr Ser
                885                 890                 895

Asp Lys Pro His Ser Ala Leu Pro Glu Glu Val Gln Glu Leu Phe Ile
            900                 905                 910

Arg Asp Tyr Gly Glu Ile Phe Asn Val Gln Leu Pro Asp Lys Glu Glu
        915                 920                 925

Arg Thr Lys Phe Phe Glu Asp Leu Ile Leu Lys Gln Ala Ala Lys Pro
    930                 935                 940

Pro Ile Ser Lys Lys Lys Ala Val Leu Gln Ala Leu Glu Val Leu Pro
945                 950                 955                 960

Val Ala Pro Pro Pro Glu Pro Arg Ser Leu Thr Ala Glu Glu Val Lys
                965                 970                 975

Arg Leu Glu Glu Gln Glu Glu Asp Thr Phe Arg Glu Leu Arg Ile Phe
            980                 985                 990

Leu Arg Asn Val Thr His Arg Leu Ala Ile Asp Lys Arg Phe Arg Val
        995                 1000                1005

Phe Thr Lys Pro Val Asp Pro Asp Glu Val Pro Asp Tyr Val Thr Val
    1010                1015                1020

Ile Lys Gln Pro Met Asp Leu Ser Ser Val Ile Ser Lys Ile Asp Leu
1025                1030                1035                1040

His Lys Tyr Leu Thr Val Lys Asp Tyr Leu Arg Asp Ile Asp Leu Ile
                1045                1050                1055

Cys Ser Asn Ala Leu Glu Tyr Asn Pro Asp Arg Asp Pro Gly Asp Arg
            1060                1065                1070

Leu Ile Arg His Arg Ala Cys Ala Leu Arg Asp Thr Ala Tyr Ala Ile
        1075                1080                1085

Ile Lys Glu Glu Leu Asp Glu Asp Phe Glu Gln Leu Cys Glu Glu Ile
    1090                1095                1100

Gln Glu Ser Arg Lys Lys Arg Gly Cys Ser Ser Ser Lys Tyr Ala Pro
1105                1110                1115                1120

Ser Tyr Tyr His Val Met Pro Lys Gln Asn Ser Thr Leu Val Gly Asp
                1125                1130                1135

Lys Arg Ser Asp Pro Glu Gln Asn Glu Lys Leu Lys Thr Pro Ser Thr
                1140                1145                1150

Pro Val Ala Cys Ser Thr Pro Ala Gln Leu Lys Arg Lys Ile Arg Lys
            1155                1160                1165

Lys Ser Asn Trp Tyr Leu Gly Thr Ile Lys Lys Arg Arg Lys Ile Ser
        1170                1175                1180

Gln Ala Lys Asp Asp Ser Gln Asn Ala Ile Asp His Lys Ile Glu Ser
1185 1190 1195 1200

Asp Thr Glu Glu Thr Gln Asp Thr Ser Val Asp His Asn Glu Thr Gly
1205 1210 1215

Asn Thr Gly Glu Ser Ser Val Glu Glu Asn Glu Lys Gln Gln Asn Ala
                1220                1225                1230

Ser Glu Ser Lys Leu Glu Leu Arg Asn Asn Ser Asn Thr Cys Asn Ile
            1235                1240                1245

Glu Asn Glu Leu Glu Asp Ser Arg Lys Thr Thr Ala Cys Thr Glu Leu
        1250                1255                1260

Arg Asp Lys Ile Ala Cys Asn Gly Asp Ala Ser Ser Ser Gln Ile Ile
1265 1270 1275 1280

His Ile Ser Asp Glu Asn Glu Gly Lys Glu Met Cys Val Leu Arg Met
1285 1290 1295

Thr Arg Ala Arg Arg Ser Gln Val Glu Gln Gln Gln Leu Ile Thr Val
                1300                1305                1310

Glu Lys Ala Leu Ala Ile Leu Ser Gln Pro Thr Pro Ser Leu Val Val
            1315                1320                1325

Asp His Glu Arg Leu Lys Asn Leu Leu Lys Thr Val Val Lys Lys Ser
        1330                1335                1340

Gln Asn Tyr Asn Ile Phe Gln Leu Glu Asn Leu Tyr Ala Val Ile Ser
1345 1350 1355 1360

Gln Cys Ile Tyr Arg His Arg Lys Asp His Asp Lys Thr Ser Leu Ile
1365 1370 1375

Gln Lys Met Glu Gln Glu Val Glu Asn Phe Ser Cys Ser Arg
1380 1385 1390

2. Procédé selon la revendication 1, dans lequel le fragment du co-modulateur comprend les acides aminés 813 à 1390 de l'ARAP11.

3. Procédé selon l'une des revendications précédentes, dans lequel le récepteur nucléaire est choisi parmi le récepteur de l'androgène, le récepteur de l'oestrogène α, le récepteur de l'oestrogène β, le récepteur de la progestérone A, le récepteur de la progestérone B, le récepteur du glucocorticoïde, le récepteur du minéralocorticoïde, le récepteur de l'hormone thyroïdienne, le récepteur de la vitamine D, le récepteur activé par le proliférateur des peroxysomes, le récepteur de l'acide rétinique, le récepteur du rétinoïde X et les récepteurs orphelins.

4. Procédé selon l'une des revendications précédentes, dans lequel les cellules sont des lignées cellulaires établies et/ou des cellules eucaryotes.

5. Procédé selon la revendication 4, dans lequel les cellules eucaryotes sont choisies parmi les cellules prostatiques, les cellules nerveuses, les cellules gliales, les fibroblastes, les cellules du sang, les ostéoblastes, les ostéoclastes, les hépatocytes, les cellules épithéliales ou les cellules musculaires.

6. Procédé selon l'une des revendications précédentes, dans lequel le vecteur est un vecteur d'expression eucaryote.

7. Procédé pour déterminer les perturbations du mécanisme de co-modulation entre des récepteurs de l'androgène et l'ARAP11, dans lequel on mesure la concentration de l'ARAP11 ou d'un fragment de ce dernier, et/ou du récepteur de l'androgène ou d'un fragment de ce dernier.

8. Procédé selon la revendication 7, dans lequel la mesure de la concentration s'effectue par analyse radio-immunologique, des essais ELISA, une immuno-coloration, une PCR par transcriptase inverse, un transfert Western, un transfert Northem, une puce à ADN ou une puce protéique.

9. Protéine ou fragment de cette dernière, présentant des propriétés de co-modulateur pour le récepteur de l'androgène, la protéine ou le fragment présentant la séquence d'acides aminés de l'ARAP11 selon la revendication 1.

10. Protéine selon la revendication 9, dans lequel le fragment comprend les acides aminés 813 à 1390.

11. ADN codant pour la protéine selon la revendication 9 ou 10, ou ADN s'hybridant à celle-ci.
